# EUROPÄISCHE PATENTSCHRIFT

(11) **EP 3 138 539 B1**
(45) Veröffentlichungstag und Bekanntmachung des Hinweises auf die Patenterteilung: **23.05.2018**
(21) Anmeldenummer: 16186759.3
(22) Anmeldetag: 01.09.2016
(51) Int. Cl.: A61F 5/01, A61F 5/058

(54) **ORTHESE MIT ORTHESENVERSCHLUSS**
ORTHOSIS WITH ORTHOSIS CLOSURE
ORTHÈSE AVEC FERMETURE D'ORTHÈSE

(30) Priorität: 03.09.2015 DE 202015006252 U
(43) Veröffentlichungstag der Anmeldung: 08.03.2017
(73) Patentinhaber: Thuasne Deutschland GmbH, 30938 Burgwedel (DE)
(72) Erfinder: SCHÖBEL, Herbert, 30938 Burgwedel (DE)
(74) Vertreter: Siekmann, Gunnar

(56) Entgegenhaltungen:
- EP-B1- 0 820 741
- US-A1- 2013 211 303
- US-A1- 2014 330 188
- US-B1- 6 893 410

## Beschreibung

Die Erfindung betrifft eine Orthese aufweisend einen Grundkörper zur Anlage um einen Körperteil mit einer ersten Kante und einer zweiten Kante sowie einen Orthesenverschluss, mit dem die erste Kante und die zweite Kante des Grundkörpers zueinander festzurrbar sind, wobei der Orthesenverschluss wenigstens einen Zugstrang umfasst, der an der ersten Kante und an der zweiten Kante des Grundkörpers befestigt ist, indem der Zugstrang mehrfach an der ersten Kante und an der zweiten Kante des Grundkörpers in Führungen geführt ist, wobei der Zugstrang durch die Führungen in einzelne Strangabschnitte unterteilt ist und der Zugstrang an den Führungen derart umgelenkt ist, dass sich wenigstens ein Teil der Strangabschnitte zwischen der ersten Kante und der zweiten Kante des Grundkörpers, diese miteinander verbindend, erstreckt, dem Zugstrang wenigstens eine Anfasslasche zum Festsetzen dieses Zugstranges an dem Grundkörper zugeordnet ist, und die Anfasslasche dabei verschiebbar zu dem Zugstrang an diesem Zugstrang befestigt ist.

Eine solche Orthese geht beispielsweise aus der US 2013/0211303 A1 hervor, bei der eine Vielzahl von Zugsträngen mit einem ersten Ende an einer ersten Kante eines Grundkörpers der Orthese und mit einem zweiten Ende an einer Anfasslasche zum Festsetzen dieser Zugstränge an dem Grundkörper der Orthese befestigt sind und an einer, der ersten Kante gegenüberliegenden, zweiten Kante des Grundkörpers jeweils durch Ösen geführt sind. Durch Ziehen an der Anfasslasche in Richtung der ersten Kante werden die beiden Kanten dann zueinander festgezurrt und durch Anlegen der Anfasslasche an dem Grundkörper fixiert.

Aus der EP 0 820 741 B1 geht eine weitere Orthese hervor, bei der Zugstränge auf ähnliche Weise an dem Grundkörper der Orthese festgesetzt werden wie bei der US 2013/0211303 A1. Im Unterschied zur US 2013/0211303 A1 ist jeder Anfasslasche jedoch nur ein Zugstrang zugeordnet, der sowohl an der ersten Kante als auch an der Anfasslasche mehrfach umgelenkt ist. Die mit einem Zugstrang aufgebrachte Zugkraft wird so auf eine größere Fläche beziehungsweise einen längeren Kantenabschnitt der ersten Kante und der zweiten Kante verteilt. Die Anfasslasche und der Zugstrang sind dabei über ein mehrere Ösen aufweisendes Halteteil miteinander verbunden, an dem einzelne Strangabschnitte des Zugstranges in einem Scheitelpunkt zusammenlaufen. Um die Orthese über die komplette Länge der ersten Kante und der zweiten Kante an einem Körperteil fixieren zu können, sind gemäß der EP 0 820 741 B1 mehrere, das heißt insbesondere wenigstens zwei, dieser mehrfach umgelenkten Zugstränge und Anfasslaschen an dem Grundkörper vorgesehen. Durch mehr als eine Anfasslasche soll vermieden werden, dass die durch die Ösen der zweiten Kante geführten Strangabschnitte des Zugstranges, die zu dem Scheitelpunkt zusammenlaufen, zu stark abgelenkt werden, wobei eine starke Ablenkung eine exakte Positionierung der Orthese an dem Körperteil erschweren und den Tragekomfort für einen Patienten verringern würde.

Die in der US 2014/0330188 A1 offenbarte Orthese besteht aus einem Grundkörper, der an ein Handgelenk angelegt werden kann. Zum Fixieren ist diesem Grundkörper ein Zugstrang zugeordnet, der über mehrere Ösen mit dem Grundkörper verbunden ist und ein als Anfasslasche ausgebildetes Zugelement aufweist. Das Zugelement ist dabei an zwei verschiedenen, zwischen Ösen des Grundkörpers ausgebildeten, Strangabschnitten des Zugstrangs befestigt und ermöglicht ein Festzurren der Orthese. Die US 2014/0330188 offenbart alle Merkmale des Oberbegriffs des Anspruchs 1. Aufgabe der Erfindung ist es eine Orthese dahingehend weiterzuentwickeln, dass diese auf einfache Weise an einem Körperteil fixierbar ist und einen über Ihre gesamte Länge gleich guten Sitz an dem Körperteil und damit einen hohen Tragekomfort gewährleistet.

Die Lösung dieser Aufgabe erfolgt mit einer Orthese mit den Merkmalen des Schutzanspruches 1. Weiterbildungen und vorteilhafte Ausgestaltungen sind in den nachgeordneten Schutzansprüchen 2 bis 15 angegeben.

Die Orthese aufweisend einen Grundkörper zur Anlage um einen Körperteil mit einer ersten Kante und einer zweiten Kante sowie einen Orthesenverschluss, mit dem die erste Kante und die zweite Kante des Grundkörpers zueinander festzurrbar sind, wobei der Orthesenverschluss wenigstens einen Zugstrang umfasst, der an der ersten Kante und an der zweiten Kante des Grundkörpers befestigt ist, indem der Zugstrang mehrfach an der ersten Kante und an der zweiten Kante des Grundkörpers in Führungen geführt ist, wobei der Zugstrang durch die Führungen in einzelne Strangabschnitte unterteilt ist und der Zugstrang an den Führungen derart umgelenkt ist, dass sich wenigstens ein Teil der Strangabschnitte zwischen der ersten Kante und der zweiten Kante des Grundkörpers, diese miteinander verbindend, erstreckt, dem Zugstrang wenigstens eine Anfasslasche zum Festsetzen dieses Zugstranges an dem Grundkörper zugeordnet ist und die Anfasslasche verschiebbar zu dem Zugstrang an diesem Zugstrang befestigt ist, zeichnet sich erfindungsgemäß dadurch aus, dass die Anfasslasche zum Festsetzen des Zugstranges an einem einzigen Strangabschnitt zwischen zwei benachbarten Führungen an den Zugstrang angreift, dass der Zugstrang mit der Anfasslasche zum Festsetzen dieses Zugstranges an dem Grundkörper derart spannbar ist, dass die erste Kante und die zweite Kante bei angelegter Orthese mit dem Zugstrang zusammenziehbar sind, und dass die ausgehend von der Anfasslasche zum Festsetzen des Zugstranges zueinander benachbarten, sich zwischen der ersten und der zweiten Kante erstreckenden Strangabschnitte zu beiden Seiten der Anfasslasche beim Festzurren des Zugstranges jeweils in einander entgegengesetzte Richtungen gezogen werden.

Aufgrund der mehrfachen Umlenkung des Zugstranges muss zudem nur wenig Zugkraft aufgewendet werden, so dass auch ältere oder geschwächte Patienten ausreichend Zugkraft zum Festzurren aufbringen können, um eine sichere Positionierung der Orthese an dem jeweiligen Körperteil zu erreichen. Die Umlenkungen des Zugstranges wirken dabei wie ein Flaschenzug. Die mehrfache Umlenkung des Zugstranges erlaubt weiterhin auch nahezu beliebig lange Kanten einer derartigen Orthese mit nur einer Anfasslasche zum Festsetzen des Zugstranges an ein Körperteil anzulegen.

Die Verschiebbarkeit der Anfasslasche zu dem Zugstrang ermöglicht dabei einen notwendigen Längenausgleich, um eine Anpassung an unterschiedliche Körperkonturen, beispielsweise dickere und dünnere Abschnitte eines Körperteils, an das die Orthese angelegt werden soll, zu erreichen. Die Anfasslasche des Zugstranges ist dabei zwischen den Führungen, die den Strangabschnitt, an den die Anfasslasche angreift, begrenzen, verschiebbar, insbesondere frei verschiebbar.

Bevorzugt sind dabei mit der Anfasslasche zum Festsetzen des Zugstranges immer alle Strangabschnitte des Zugstranges gemeinsam betätigbar. Durch Ziehen der Anfasslasche und Festsetzen dieser an dem Grundkörper der Orthese kann die Orthese dabei auf einfache Weise einhändig an ein Körperteil angelegt und über die gesamte Länge der ersten und zweiten Kante gleichmäßig festgezurrt werden. Das Anlegen der Orthese kann damit von einem Patienten ohne Hilfe einer weiteren Person erfolgen. Ein Nachjustieren entfällt dabei vorteilhafterweise.

Eine besonders einfache Handhabung der Anfasslasche kann dadurch erreicht sein, dass die Anfasslasche wenigstens eine Klettfläche aufweist, die mit dem Grundkörper verhakbar ist. Die mit dem Grundkörper verhakbare Klettfläche kann sich dabei über eine gesamte Seite der Anfasslasche, oder nur einen Teilbereich einer Seite der Anfasslasche erstrecken. bevorzugtes Material für die Anfasslasche ist das gleiche Material wie für den Grundkörper, um ein optisch einheitliches und damit hochwertigeres Aussehen zu gewährleisten.

In weiterer Ausgestaltung kann die Anfasslasche ein Stoffstück sein, welches an einem Strangabschnitt des Zugstranges, insbesondere einem einzigen Strangabschnitt des Zugstranges, an diesem befestigt ist. Weiter kann an der Anfasslasche, insbesondere einem Ende der Anfasslasche, eine Zugstranghalterung ausgebildet sein. Über die Zugstranghalterung ist die Anfasslasche dann mit dem Zugstrang verbunden. Hierzu kann die Zugstranghalterung als hülsenförmige Führung ausgebildet sein. Innerhalb der hülsenförmigen Führung ist der Zugstrang vorteilhafterweise verschiebbar geführt.

Um ein Verhaken der Klettfläche der Anfasslasche an dem Grundkörper zu ermöglichen, kann der Grundkörper entweder bestimmte Klettbereiche aufweisen, die aus einem geeigneten Material zum Verhaken der Klettfläche der Anfasslasche bestehen, oder außenseitig vollflächig aus geeignetem Material gefertigt sein, so dass die Anfasslasche beliebig an dem Grundkörper festgelegt werden kann und immer die für den Patienten optimale Positionierung ermöglicht ist.

Eine über die gesamte Länge der zueinander festzurrbaren ersten und zweiten Kante des Grundkörpers der Orthese möglichst gleichmäßige Spannkraft kann dadurch erreicht werden, dass die Anfasslasche in Längserstreckung der ersten Kante und der zweiten Kante des Grundkörpers mittig zwischen den Führungen und den einzelnen Strangabschnitten an den Zugstrang angreift. Beim Festzurren des Zugstranges werden dann idealerweise die jeweils in Richtung der äußeren Enden abgehenden Strangabschnitte des Zugstranges möglichst gleich weit bewegt.

Eine besonders günstige Verteilung der einzelnen Strangabschnitte zwischen der ersten Kante und der zweiten Kante des Grundkörpers kann dadurch sichergestellt sein, dass sich jeder zweite Strangabschnitt zwischen der ersten Kante und der zweiten Kante, diese miteinander verbindend, erstreckt. Zwischen den die erste Kante und die zweite Kante miteinander verbindenden Strangabschnitten des Zugstranges können dann parallel zu wenigstens einer der Kanten verlaufende Strangabschnitte ausgebildet sein. Der Zugstrang ist dann an den beiden Kanten des Grundkörpers aufeinander folgend jeweils immer durch zwei der ersten Kante oder zwei der zweiten Kante zugeordnete Führungen geführt, bevor sich ein Strangabschnitt von der einen Kante zu der anderen Kante erstreckt. Lediglich an den äußeren Enden der Kanten des Grundkörpers können in bevorzugter Ausgestaltung einzelne Führungen vorzusehen sein. An den Führungen erfolgt dann bevorzugt jeweils eine Umlenkung des Zugstranges um ungefähr 90°, das heißt zwischen 80° und 100°, insbesondere zwischen 85° und 95°, insbesondere genau 90°, so dass jeder zweite Strangabschnitt im Wesentlichen parallel zueinander verläuft.

Durch verändern der Abstände der Führungen, insbesondere der paarweise an einer der Kanten angeordneten Führungen, zueinander kann zudem auf einfache Weise ein gewünschter Abstand der sich zwischen der ersten und der zweiten Kante erstreckenden, diese miteinander verbindenden, Strangabschnitte vorgesehen werden, der einen optimalen Sitz der Orthese an dem Körperteil sicherstellt. Durch Kombination unterschiedlicher Abstände der Führungen miteinander kann weiterhin eine besonders gute Anpassung an Konturen bestimmter Körperteile erfolgen. In einzelnen Bereichen können die Abstände der die erste Kante und die zweite Kante miteinander verbindenden Strangabschnitte dann enger sein und in anderen Bereichen können die Strangabschnitte mit größerem Abstand zueinander angeordnet sein. Bevorzugt sind die die erste Kante und die zweite Kante miteinander verbindenden Strangabschnitte in Abständen von weniger als 10 cm, insbesondere in Abständen von weniger als 7 cm, insbesondere in Abständen zwischen 3 cm und 5 cm, zueinander angeordnet.

Sind einzelne Führungen vorgesehen, an die jeweils zwei sich zwischen der ersten Kante und der zweiten Kante erstreckende Strangabschnitte ansetzen, sind diese Strangabschnitte in jedem Fall diagonal zu der ersten Kante und der zweiten Kante ausgerichtet. Die Strangabschnitte verlaufen dann "zickzack"-förmig zueinander.

Der Zugstrang selbst kann nach einer Weiterbildung eine Ringschnur sein, die an den in Längserstreckung äußeren Enden der ersten Kante oder der zweiten Kante parallel zu der jeweiligen Kante zum jeweils anderen äußeren Ende rückgeführt ist. Eine solche Ringschnur als Zugstrang ermöglicht eine nochmals verbesserte Anpassung und einen gleichmäßigeren Anpressdruck der Orthese an das jeweilige Körperteil, indem ein Längenausgleich zwischen den sich jeweils von der Anfasslasche zu den äußeren Enden der ersten und der zweiten Kante des Grundkörpers erstreckenden Strangabschnitten gewährleistet ist. Die Orthese kann dann auch an ein beispielsweise konisch geformtes Körperteil angepasst werden, wobei in einem dickeren Bereich des Körperteils mehr Stranglänge benötigt wird als in einem dünneren Bereich des Körperteils, um einen gleichmäßigen Anpressdruck an das Körperteil im dickeren Bereich und im dünneren Bereich zu erreichen. Der Längenausgleich des Zugstranges erfolgt dabei über den an den in Längserstreckung äußeren Enden der ersten Kante oder der zweiten Kante parallel zu der jeweiligen Kante zum jeweils anderen äußeren Ende rückgeführten Strangabschnitt der Ringschnur, der sich beim Festzurren entsprechend der für den jeweiligen Längenausgleich benötigten Stranglänge zu dem dickeren Bereich des Körperteils hin verschiebt.

Nach einer Weiterbildung kann die Rückführung des Zugstranges abgedeckt zwischen zwei Lagen des Grundkörpers angeordnet sein. An den äußeren Enden der ersten Kante oder der zweiten Kante ist dann jeweils eine Öffnung innerhalb einer äußeren Lage des Materials des Grundkörpers ausgebildet. Um ein Ausreißen dieser Öffnungen in dem Grundkörper beim Festzurren der Orthese zu verhindern, kann jeder dieser Öffnungen noch eine der Führungen zugeordnet sein, die die Zugkräfte beim Spannen des Zugstranges aufnimmt. Bei einer offenliegenden, außenseitig an dem Grundkörper angeordneten Rückführung sind diese Führungen zwingend erforderlich.

Alternativ zu einer Ringschnur kann als Zugstrang auch ein endlicher Strang vorgesehen sein, der mit seinen Enden an den äußeren Enden der ersten Kante oder der zweiten Kante festgelegt ist. Die Rückführung entlang einer der Kanten des Grundkörpers entfällt dann, so dass eine einfachere Fertigung ermöglicht ist.

Die Führungen des Zugstranges sind gemäß einer ersten Ausführung durch in dem Grundkörper angeordneten Ausnehmungen, insbesondere durch das Material des Grundkörpers hindurchführende Ausnehmungen, gebildet. Bei einer Ringschnur bilden die jeweils äußersten, den äußeren Enden der ersten Kante oder der zweiten Kante zugeordneten Ausnehmungen dann jeweils die Öffnungen der abgedeckten Rückführung. Die Ausnehmungen können in weiterer Ausgestaltung auch zusätzlich verstärkt sein, beispielsweise durch Metallringe, die den Rand der Ausnehmungen einfassen. Um die Führungen vollständig außenseitig an dem Grundkörper anzuordnen, eignen sich gemäß einer anderen, zweiten Ausführung außenseitig an dem Grundkörper angeordnete, insbesondere angenähte, Laschen als Führungen des Zugstranges. Jede Lasche ist dabei mit zwei einander entgegengesetzten Enden an dem Grundkörper festgenäht und bildet eine Öse aus, durch die der Zugstrang hindurchführbar ist. Innenseitig ist der Grundkörper damit frei von Teilen des Orthesenverschlusses, so dass ein besonders angenehmes Tragegefühl ermöglicht ist.

Eine Erhöhung des Tragekomforts der Orthese ist zudem dadurch erreichbar, dass zwischen der ersten und der zweiten Kante eine Stoffbahn zwischen den beiden Kanten angeordnet ist, die insbesondere mit der ersten Kante und der zweiten Kante verbunden ist. Diese verhindert, dass die festgezurrten Strangabschnitte in das zu behandelnde Körperteil des Patienten einschneiden. Weiterhin können auch mehr als eine Anfasslasche vorgesehen sein, um insbesondere eine bessere Passform bei besonders langen Kanten zu verbessern.

In weiterer Ausgestaltung ist die Orthese eine Handgelenkorthese. Diese kann in den Grundkörper eingearbeitet wenigstens eine Schiene aufweisen, die vorteilhafterweise parallel zu der ersten und der zweiten Kante zwischen diesen angeordnet ist, insbesondere zwischen zwei Lagen des Grundkörpers angeordnet ist, insbesondere zwischen den zwei Lagen des Grundkörpers angeordnet ist, zwischen denen auch die Rückführung des Zugstrangs angeordnet ist.
Um die Orthese sowohl am linken als auch am rechten Handgelenk tragen zu können, kann diese weiterhin einen symmetrischen Grundkörper aufweisen, wobei die Schiene dann mittig zwischen der ersten Kante und der zweiten Kante angeordnet ist. Weiter können in dem Grundkörper Ausnehmungen für einen rechten Daumen und einen linken Daumen oder einen rechten Daumen oder einen linken Daumen vorgesehen sein, wobei die Ausführung mit zwei Ausnehmungen für beide Handgelenke, das heißt linkes Handgelenk und rechtes Handgelenk, geeignet ist. Bei angelegter Orthese ist die Schiene dann innenseitig an der Handinnenfläche und dem sich an die Handinnenfläche anschließenden Teil des Unterarms anliegend angeordnet. Die erste Kante und die zweite Kante liegen bei angelegter Orthese an dem Handrücken und dem sich an den Handrücken anschließenden Teil des Unterarms an. Beim Zusammenziehen der beiden Kanten des Grundkörpers beziehungsweise Festzurren des Zugstranges werden die erste Kante und die zweite Kante dann auf dem Handrücken zueinander zusammengezogen.

In weiterer Ausgestaltung sind jeder der beiden Kanten der Orthese sechs Führungen zugeordnet. An der ersten Kante sind dann ausgehend von einem distalen Ende der Orthese beziehungsweise einer Hand des Patienten bei angelegter Orthese die erste und zweite Führung sowie die fünfte und sechste Führung und an der zweiten Kante die zweite und dritte Führung sowie die vierte und fünfte Führung jeweils ein Paar ausbildend. Ein Paar ausbildend heißt, dass der Zugstrang durch diese Führungen aufeinanderfolgend an einer der Kanten hindurchgeführt ist. Zwischen der dritten und vierten Führung der ersten Kante greift die Anfasslasche des Zugstranges an den Zugstrang an. Die ausgehend vom distalen Ende der Orthese erste und sechste Führung der zweiten Kante bilden jeweils äußere Endpunkte des Orthesenverschlusses und begrenzen die Rückführung des Zugstranges. Die Orthese weist damit insgesamt sechs sich zwischen der ersten Kante und der zweiten Kante, diese miteinander verbindend, erstreckende Strangabschnitte auf.

Ein Ausführungsbeispiel der Erfindung ist in der Zeichnung dargestellt. Es zeigen:
- Figur 1:: eine erste Draufsicht auf eine erfindungsgemäße Orthese;
- Figur 2:: eine zweite Draufsicht auf die Orthese gemäß Figur 1;
- Figur 3:: eine dritte Draufsicht auf die Orthese gemäß Figur 1 und 2;
- Figur 4:: eine vierte Draufsicht auf die Orthese gemäß Figur 1 bis 3;
- Figur 5:: eine fünfte Draufsicht auf die Orthese gemäß Figur 1 bis 4;
- Figur 6:: eine Seitenansicht der Orthese gemäß Figur 5;
- Figur 7:: eine sechste Draufsicht auf die Orthese gemäße Figur 1 bis 6; und
- Figur 8:: eine siebte Draufsicht auf die Orthese gemäß Figur 1 bis 7.

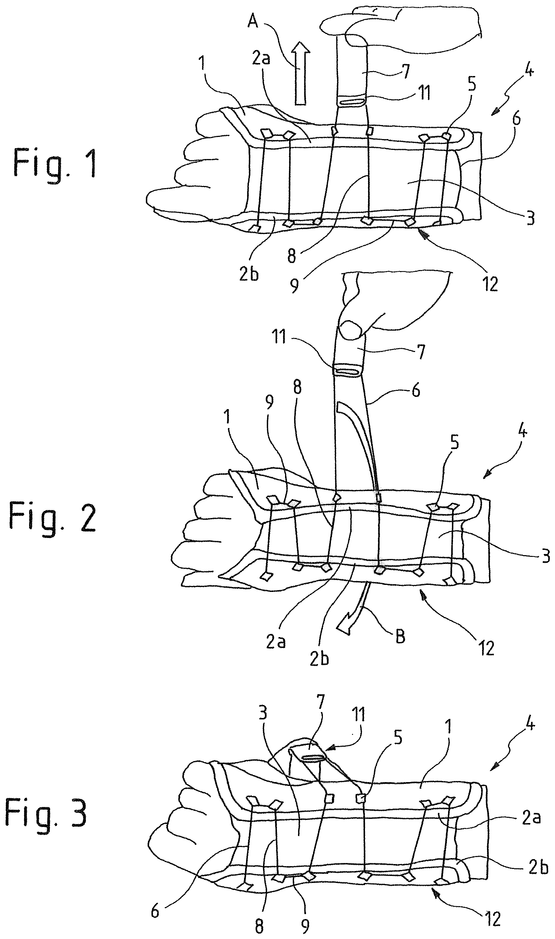

In Figur 1 ist eine erfindungsgemäße, um ein Handgelenk und einen Unterarm positionierte Orthese aufgezeigt. Diese besteht aus einem an dem Handgelenk anliegenden Grundkörper 1, der parallel seiner Längsachse von einer ersten Kante 2a und einer zweiten Kante 2b begrenzt ist. Die erste Kante 2a und die zweite Kante 2b, sind an dem Handrücken sowie in dessen Verlängerung an den sich anschließenden Teil des Unterarms in Anlage gebracht, wobei der Grundkörper 1 das Handgelenk umschließt. Im Bereich des Handrückens sind die Kanten 2a und 2b des Grundkörpers 1 zudem über eine Stoffbahn 3 miteinander verbunden.

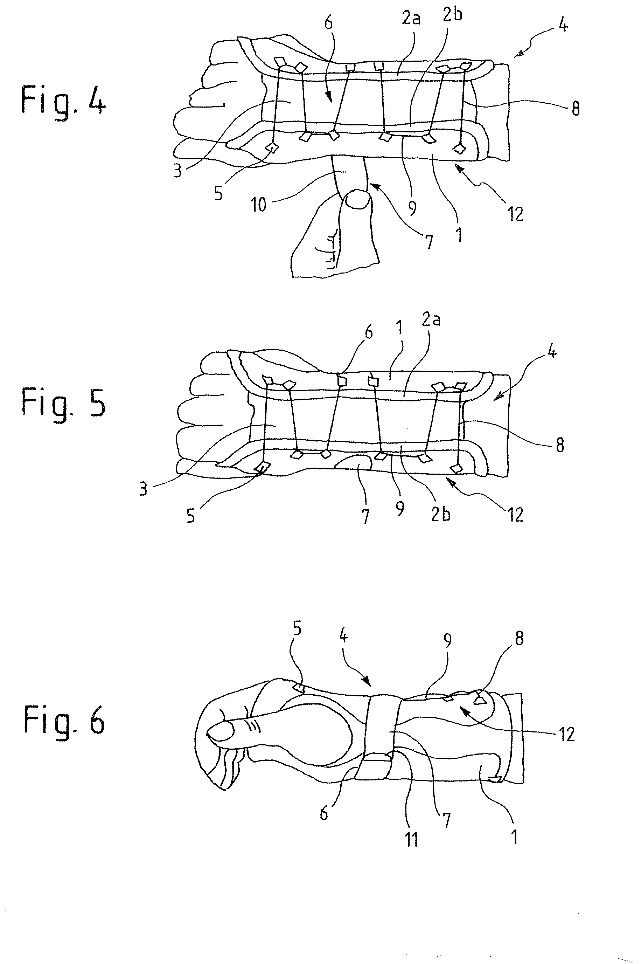

Weiter weist die Orthese einen Orthesenverschluss 4 auf. Dieser Orthesenverschluss 4 setzt sich aus an der ersten Kante 2a und an der zweiten Kante 2b angeordneten Führungen 5, einem in den Führungen 5 geführten Zugstrang 6 sowie einer Anfasslasche 7 zum Festsetzen des Zugstrangs 6 an dem Grundkörper 1 zusammen. Wie aus Figur 1 weiter zu entnehmen ist, sind die Führungen 5 dabei immer paarweise angeordnet, wobei der Zugstrang 6 durch die Führungen 5 in einzelne Strangabschnitte 8, 9 unterteilt ist. Diese Strang-abschnitte 8, 9 erstrecken sich jeweils zwischen zwei der Führungen 5, wobei sich die Strangabschnitte 8 zwischen den beiden Kanten 2a, 2b, diese miteinander verbindend, erstrecken und die Strangabschnitte 9 jeweils im Wesentlichen parallel zu wenigstens einer der jeweiligen Kanten 2a, 2b ausgerichtet sind. Jeder der beiden Kanten 2a, 2b sind sechs Führungen 5 zugeordnet. An der ersten Kante 2a sind ausgehend vom distalen Ende der Orthese beziehungsweise der Hand des Patienten die erste und zweite Führung 5 sowie die fünfte und sechste Führung 5 und an der zweiten Kante 2b die zweite und dritte Führung 5 sowie die vierte und fünfte Führung 5 jeweils ein Paar ausbildend. Ein Paar ausbildend heißt, dass der Zugstrang 6 durch diese Führungen 5 aufeinanderfolgend an einer der Kanten hindurchgeführt ist. Zwischen der dritten und vierten Führung 5 der ersten Kante 2a greift die Anfasslasche 7 zum Festsetzen des Zugstranges 6 an den Zugstrang 6 an. Diese Anfasslasche 7 mit einer in Figur 4 zu sehenden, einseitigen Klettfläche 10 ist außenseitig an dem Grundkörper 1 festsetzbar. Die Anfasslasche 7 zum Festsetzen des Zugstranges 6 ist weiter über eine Zugstranghalterung 11 mit dem Zugstrang 6 verbunden, wobei die Zugstranghalterung 11 eine hülsenförmige Führung in welcher der Zugstrang 6 verschiebbar geführt ist umfasst.

Die ausgehend vom distalen Ende der Orthese erste und sechste Führung an der zweiten Kante 2b stellen einander gesetzte Enden einer Rückführung 12 des als Ringschnur ausgebildeten Zugstranges 6 dar. Diese Rückführung 12 ist zwischen zwei Lagen des Grundkörpers 1 abgedeckt angeordnet und erstreckt sich von dem distalen Ende der Orthese bis zu einem proximalen Ende der Orthese.

Die weiteren Figuren 2 bis 5 sowie 7 und 8 zeigen jeweils identische Ansichten der Orthese gemäß Figur 1, wobei gleiche Teile mit gleichen Bezugszeichen versehen sind.

Mit den Figuren 1 bis 5 wird das Festzurren der Orthese an dem Handgelenk aufgezeigt. Die Anfasslasche 7 zum Festsetzen des Zugstranges 6 wird in Figur 1 mit einer Hand ergriffen und, wie in Figur 2 zu sehen ist, von der Orthese in Richtung Pfeil A weggezogen, so dass der Zugstrang 6 gespannt wird. Dieser gleitet dabei durch die Führungen 5, wobei die erste Kante 2a und die zweite Kante 2b aufeinander zu bewegt werden und die Strangabschnitte 8 verkürzt werden. Dabei wird ein über die Länge der ersten Kante 2a und die Länge der zweiten Kante 2b gleichmäßiger Anpressdruck der Orthese an dem Handgelenk erreicht. Im nachfolgenden Schritt wird die Anfasslasche 7 in Richtung Pfeil B um die Orthese gelegt, wie dies in Figur 3 und Figur 4 aufgezeigt ist. Der Zugstrang 6 bleibt dabei durchgehend gespannt bis die Anfasslasche 7, wie in Figur 4 dargestellt, mit der Klettfläche 10 an den Grundkörper 1 angedrückt wird.

Figur 5 und Figur 6 zeigen schließlich die fertig angelegte Orthese aus zwei Richtungen, wobei aus Figur 6 insbesondere die seitliche Lage der an den Grundkörper 1 angedrückten Anfasslasche 7 hervorgeht.

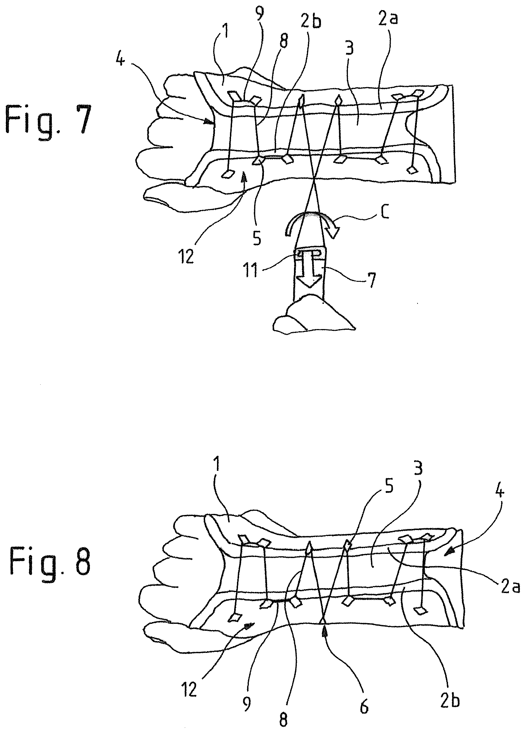

In Figur 7 und 8 ist eine alternative Befestigung der Anfasslasche 7 an dem Grundkörper 1 aufgezeigt. Dabei wird die Anfasslasche 7 entgegen Figur 1 bis 6 um 180° in Richtung Pfeil C um seine Längsachse gedreht, in entgegengesetzter Richtung durch Wegziehen der Anfasslasche 7 von dem Grundkörper 1 gespannt, um die Orthese gewickelt und mit der Klettfläche 10, an den Grundkörper 1 angedrückt.

Alle in der vorstehenden Beschreibung und in den Ansprüchen genannten Merkmale sind in einer beliebigen Auswahl mit den Merkmalen des unabhängigen Anspruchs kombinierbar. Die Offenbarung der Erfindung ist somit nicht auf die beschriebenen beziehungsweise beanspruchten Merkmalskombinationen beschränkt, vielmehr sind alle im Rahmen der Erfindung sinnvollen Merkmals-kombinationen als offenbart zu betrachten.

## Patentansprüche

1. Orthese aufweisend einen Grundkörper (1) zur Anlage um ein Körperteil mit einer ersten Kante (2a) und einer zweiten Kante (2b) sowie einen Orthesenverschluss (4), mit dem die erste Kante (2a) und die zweite Kante (2b) des Grundkörpers (1) zueinander festzurrbar sind, wobei der Orthesenverschluss (4) wenigstens einen Zugstrang (6) umfasst, der an der ersten Kante (2a) und an der zweiten Kante (2b) des Grundkörpers (1) befestigt ist, indem der Zugstrang (6) mehrfach an der ersten Kante (2a) und an der zweiten Kante (2b) des Grundkörpers (1) in Führungen (5) geführt ist, wobei der Zugstrang (6) durch die Führungen (5) in einzelne Strangabschnitte (8, 9) unterteilt ist und der Zugstrang (6) an den Führungen (5) derart umgelenkt ist, dass sich wenigstens ein Teil der Strangabschnitte (8) zwischen der ersten Kante (2a) und der zweiten Kante (2b) des Grundkörpers (1), diese miteinander verbindend, erstreckt, dem Zugstrang (6) wenigstens eine Anfasslasche (7) zum Festsetzen dieses Zugstranges (6) an dem Grundkörper (1) zugeordnet ist, und die Anfasslasche (7) verschiebbar zu dem Zugstrang (6) an diesem Zugstrang (6) befestigt ist,
**dadurch gekennzeichnet,**
**dass** die Anfasslasche (7) zum Festsetzen des Zugstranges (6) an einem einzigen Strangabschnitt (9) zwischen zwei benachbarten Führungen (5) an den Zugstrang (6) angreift,
**dass** der Zugstrang (6) mit der Anfasslasche (7) zum Festsetzen dieses Zugstranges (6) an dem Grundkörper (1) derart spannbar ist, dass die erste Kante (2a) und die zweite Kante (2b) bei angelegter Orthese mit dem Zugstrang (6) zusammenziehbar sind, und
**dass** die ausgehend von der Anfasslasche (7) zum Festsetzen des Zugstranges (6) zueinander benachbarten, sich zwischen der ersten Kante (2a) und der zweiten Kante (2b) erstreckenden Strangabschnitte (8) zu beiden Seiten der Anfasslasche (7) beim Festzurren des Zugstranges (6) jeweils in einander entgegengesetzte Richtungen gezogen werden.

2. Orthese nach Anspruch 1, **dadurch gekennzeichnet, dass** mit der Anfasslasche (7) zum Festsetzen des Zugstranges (6) immer alle Strangabschnitte (8, 9) des Zugstranges (6) gemeinsam betätigbar sind.

3. Orthese nach einem der Ansprüche 1 oder 2, **dadurch gekennzeichnet, dass** die Anfasslasche (7) wenigstens eine Klettfläche (10) aufweist, die mit dem Grundkörper (1) verhakbar ist.

4. Orthese nach einem der Ansprüche 1 bis 3, **dadurch gekennzeichnet, dass** dem Zugstrang genau eine Anfasslasche zugeordnet ist.

5. Orthese nach einem der Ansprüche 1 bis 4, **dadurch gekennzeichnet, dass** die Anfasslasche (7) in Längserstreckung der ersten Kante (2a) und der zweiten Kante (2b) des Grundkörpers (1) mittig zwischen den Führungen (5) an dem Zugstrang (6) angreift.

6. Orthese nach einem der Ansprüche 1 bis 5, **dadurch gekennzeichnet, dass** sich jeder zweite Strangabschnitt (8) zwischen der ersten Kante (2a) und der zweiten Kante (2b), diese miteinander verbindend, erstreckt.

7. Orthese nach einem der Ansprüche 1 bis 6, **dadurch gekennzeichnet, dass** zwischen den die erste Kante (2a) und die zweite Kante (2b) miteinander verbindenden Strangabschnitten (8) parallel zu wenigstens einer der Kanten (2a, 2b) verlaufende Strangabschnitte (9) ausgebildet sind.

8. Orthese nach einem der Ansprüche 1 bis 7, **dadurch gekennzeichnet, dass** die Strangabschnitte "zickzack"-förmig zueinander verlaufen.

9. Orthese nach einem der Ansprüche 1 bis 8, **dadurch gekennzeichnet, dass** der Zugstrang (6) eine Ringschnur ist, die an den in Längserstreckung äußeren Enden der ersten Kante (2a) oder der zweiten Kante (2b) parallel zu der jeweiligen Kante (2a, 2b) zum jeweils anderen äußeren Ende rückgeführt ist.

10. Orthese nach Anspruch 9, **dadurch gekennzeichnet, dass** die Rückführung des Zugstrangs (6) abgedeckt zwischen zwei Lagen des Grundkörpers (1) erfolgt.

11. Orthese nach einem der Ansprüche 1 bis 8, **dadurch gekennzeichnet, dass** der Zugstrang (6) mit seinen Enden an den äußeren Enden der ersten Kante (2a) oder der zweiten Kante (2b) festgelegt ist.

12. Orthese nach einem der Ansprüche 1 bis 11, **dadurch gekennzeichnet, dass** die Führungen (5) des Zugstrangs (6) durch in dem Grundkörper (1) angeordnete Ausnehmungen gebildet sind.

13. Orthese nach einem der Ansprüche 1 bis 12, **dadurch gekennzeichnet, dass** diese eine Handgelenkorthese ist.

14. Orthese nach Anspruch 13, **dadurch gekennzeichnet, dass** in dem Grundkörper (1) Ausnehmungen für einen rechten und einen linken Daumen vorgesehen sind.

15. Orthese nach einem der Ansprüche 13 oder 14, **dadurch gekennzeichnet, dass** die erste Kante (2a) und die zweite Kante (2b) bei angelegter Orthese an dem Handrücken anliegen.

## Claims

1. Orthosis comprising a base body (1) for placement around a body part, having a first edge (2a) and a second edge (2b), and an orthosis closure (4) with which the first edge (2a) and the second edge (2b) of the base body (1) can be attached tightly to each other, wherein the orthosis closure (4) comprises at least one drawstring (6) which is attached to the first edge (2a) and to the second edge (2b) of the base body (1) by passing the drawstring (6) through multiple guides (5) on the first edge (2a) and on the second edge (2b) of the base body (1), wherein the drawstring (6) is divided into individual string sections (8, 9) by the guides (5) and the drawstring (6) is redirected by the guides (5) in such manner that at least some of the string sections (8) extend between the first edge (2a) and the second edge (2b) of the base body (1) binding them to each other, at least one gripping strap (7) is associated with the drawstring (6) for securing said drawstring (6) on the base body (1), and the gripping strap (7) is fastened to the drawstring (6) in such manner as to be displaceable relative to said drawstring (6);
**characterized in that**
the gripping strap (7) engages with the drawstring (6) at a single string section (9) between two adjacent guides (5) for securing the drawstring (6),
that the drawstring (6) can be tightened with the gripping strap (7) for securing said drawstring (6) on the base body (1) in such manner that the first edge (2a) and the second edge (2b) can be drawn together by means of the drawstring (6) when the orthosis is in place, and that the string sections (8) adjacent to each other and extending from the gripping strap (7) between the first edge (2a) and the second edge (2b) on both sides of the gripping strap (7) are drawn towards each other in opposite directions when tightening the drawstring (6) in order to secure the drawstring (6).

2. Orthosis according to Claim 1, **characterized in that** all of the string sections (8, 9) of the drawstring (6) are always be actuated together with the gripping strap (7) for securing the drawstring (6).

3. Orthosis according to Claim 1 or 2, **characterized in that** the gripping strap (7) has at least one hook- and-loop surface (10) which can be hooked to the base body (1).

4. Orthosis according to any one of Claims 1 to 3, **characterized in that** exactly one grippping strap is attached to the drawstring.

5. Orthosis according to any one of Claims 1 to 4, **characterized in that** the gripping strap (7) engages in the middle of the drawstring (6) between the guides (5) in the lengthwise extension of the first edge (2a) and the second edge (2b) of the base body (1) .

6. Orthosis according to any one of Claims 1 to 5, **characterized in that** every second string section (8) extends between the first edge (2a) and the second edge (2b), connecting them to each other.

7. Orthosis according to any one of Claims 1 to 6, **characterized in that** string sections (9) extending parallel to at least one of the edges (2a, 2b) are formed between the string sections (8) which connect the first edge (2a) and the second edge (2b) to each other.

8. Orthosis according to any one of Claims 1 to 7, **characterized in that** the string sections extend in a "zigzag" pattern relative to each other.

9. Orthosis according to any one of Claims 1 to 8, **characterized in that** the drawstring (6) is a ring cord which is routed along the outer ends of the first edge (2a) or the second edge (2b) parallel to the respective edge (2a, 2b) thereof and back to the respective other outer end.

10. Orthosis according to Claim 9, **characterized in that** the return of the drawstring (6) covered by two layers of the base body (1).

11. Orthosis according to any one of Claims 1 to 8, **characterized in that** the ends of the drawstring (6) are secured to the outer ends of the first edge (2a) or the second edge (2b).

12. Orthosis according to any one of Claims 1 to 11, **characterized in that** the guides (5) of the drawstring (6) are formed by recesses arranged in the base body (1).

13. Orthosis according to any one of Claims 1 to 12, **characterized in that** it is a wrist orthosis.

14. Orthosis according to Claim 13, **characterized in that** recesses are provided in the base body (1) for a right and a left thumb.

15. Orthosis according to either of Claims 13 or 14, **characterized in that** the first edge (2a) and the second edge (2b) lie flush against the back of the hand when the orthosis is in place.

## Revendications

1. Orthèse présentant un corps de base (1) destiné à se poser autour d'une partie de corps, comprenant un premier bord (2a) et un second bord (2b) ainsi qu'une fermeture d'orthèse (4), avec laquelle le premier bord (2a) et le second bord (2b) du corps de base (1) peuvent être amarrés fixement, dans laquelle la fermeture d'orthèse (4) comprend au moins un cordon d'étirement (6) qui est fixé sur le premier bord (2a) et le second bord (2b) du corps de base (1), en ce que le cordon d'étirement (6) est dirigé plusieurs fois dans des guides (5) sur le premier bord (2a) et sur le second bord (2b) du corps de base (1), dans lequel le cordon d'étirement (6) est divisé en tronçons de cordon (8, 9) individuels par les guides (5) et le cordon d'étirement (6) est ainsi dévié sur les guides (5) qu'au moins une partie des tronçons d'étirement (8) s'étend entre le premier bord (2a) et le second bord (2b) du corps de base (1), en reliant ceux-ci entre eux, au moins une sangle de contact (7) pour fixer ce cordon d'étirement (6) sur le corps de base (1) correspond au cordon d'étirement (6), et la sangle de contact (7) et fixée de manière mobile au cordon d'étirement (6) sur ce même cordon d'étirement (6),
**caractérisée en ce que**
la sangle de contact (7) pour fixer le cordon d'étirement (6) s'engage sur le cordon d'étirement (6) sur un seul tronçon de cordon (9) entre deux guides (5) adjacents,
que le cordon d'étirement (6) peut être ainsi tendu avec la sangle de contact (7) pour fixer ce cordon d'étirement (6) sur le corps de base (1) que le premier bord (2a) et le second bord (2b) peuvent être tirés conjointement avec le cordon d'étirement (6) lorsque l'orthèse est en place,
que les tronçons de cordon (8) adjacents, partant de la sangle de contact (7) pour fixer le cordon d'étirement (6), s'étendant entre le premier bord (2a) et le second bord (2b), peut être tiré des deux côtés de la sangle de contact (7) lorsque le cordon d'étirement (6) est amarré, respectivement dans des directions opposées entre elles.

2. Orthèse selon la revendication 1, **caractérisée en ce qu'**avec la sangle de contact (7) pour fixer le cordon d'étirement (6), tous les tronçons de cordon (8, 9) du cordon d'étirement (6) sont toujours actionnables conjointement.

3. Orthèse selon la revendication 1 ou 2, **caractérisée en ce que** la sangle de contact (7) présente au moins une surface de fermeture par boucles/crochets (10) avec laquelle le corps de base (1) peut se crocheter.

4. Orthèse selon l'une des revendications 1 à 3, **caractérisée en ce que** précisément une sangle de contact correspond au cordon d'étirement.

5. Orthèse selon l'une des revendications 1 à 4, **caractérisée en ce que** la sangle de contact (7) s'engage sur le cordon d'étirement (6) au centre entre les guides (5) dans l'allongement longitudinal du premier bord (2a) et du second bord (2b) du corps de base (1).

6. Orthèse selon l'une des revendications 1 à 5, **caractérisée en ce qu'**un tronçon d'étirement (8) sur deux s'étend entre le premier bord (2a) et le second bord (2b) en reliant ceux-ci entre eux.

7. Orthèse selon l'une des revendications 1 à 6, **caractérisée en ce que** des tronçons d'étirement (9) passant parallèlement à au moins un des bords (2a, 2b) sont formés entre les tronçons d'étirement (8) reliant entre eux le premier bord (2a) et le second bord (2b).

8. Orthèse selon l'une des revendications 1 à 7, **caractérisée en ce que** les tronçons d'étirement forment un tracé en zigzag entre eux.

9. Orthèse selon l'une des revendications 1 à 8, **caractérisée en ce que** le cordon d'étirement (6) est un cordon annulaire qui est ramené sur les extrémités extérieures dans l'allongement longitudinal du premier bord (2a) ou du second bord (2b) parallèlement au bord (2a, 2b) respectif, en direction de l'autre extrémité extérieure.

10. Orthèse selon la revendication 9, **caractérisée en ce que** le retour du cordon d'étirement (6) s'effectue couvert entre deux couches du corps de base (1).

11. Orthèse selon l'une des revendications 1 à 8, **caractérisée en ce que** le cordon d'étirement (6) est fixé par ses extrémités aux extrémités extérieures du premier bord (2a) ou du second bord (2b).

12. Orthèse selon l'une des revendications 1 à 11, **caractérisée en ce que** les guides (5) du cordon d'étirement (6) sont formés par des évidements disposés dans le corps de base (1).

13. Orthèse selon l'une des revendications 1 à 12, **caractérisée en ce qu'**il s'agit d'une orthèse de poignet.

14. Orthèse selon la revendication 13, **caractérisée en ce que** des évidements pour un pouce droit et un pouce gauche sont prévus dans le corps de base (1).

15. Orthèse selon l'une des revendications 13 ou 14, **caractérisée en ce que** le premier bord (2a) et le second bord (2b) reposent sur le dos de la main lorsque l'orthèse est en place.
